# EUROPÄISCHE PATENTSCHRIFT

(11) **EP 2 024 149 B1**
(45) Veröffentlichungstag und Bekanntmachung des Hinweises auf die Patenterteilung: **25.01.2012**
(21) Anmeldenummer: 06722859.3
(22) Anmeldetag: 11.05.2006
(51) Int. Cl.: B26F 1/38, A61F 13/00, B26F 1/44

(54) **VORRICHTUNG ZUM ROTATIVEN STANZEN VON STANZSTÜCKEN DEFINIERTER GEOMETRIE UND GRÖSSE AUS EINEM FLÄCHENGEBILDE UND DEREN VERWENDUNG**
DEVICE FOR THE ROTARY STAMPING OF STAMPED PIECES OF DEFINED GEOMETRY AND SIZE FROM A PLANAR STRUCTURE AND ITS USE
DISPOSITIF POUR L'ESTAMPAGE ROTATIF DE PIÈCES DÉCOUPÉES DE GÉOMÉTRIE ET DE TAILLE DÉFINIES CONSTITUÉ D'UNE STRUCTURE PLATE ET SON UTILISATION

(43) Veröffentlichungstag der Anmeldung: 18.02.2009
(73) Patentinhaber: Märdian Werkzeug- und Maschinenbau GmbH, 66999 Hinterweidenthal (DE)
(72) Erfinder: MÄRDIAN, Franz-Josef, 66999 Hinterweidenthal (DE)
(74) Vertreter: Wiedemann, Markus
(86) Internationale Anmeldenummer: PCT/DE2006/000811
(87) Internationale Veröffentlichungsnummer: WO 2007/131460

(56) Entgegenhaltungen:
- EP-A- 0 207 442
- US-A- 104 442
- US-A- 4 108 033
- US-A- 4 862 574

## Beschreibung

Die Erfindung betrifft eine Vorrichtung zum rotativen Stanzen von Stanzstücken definierter Geometrie und Größe aus einem Flächengebilde sowie eine Verwendung der Vorrichtung gemäß den Ansprüchen 1 und 15.

In vielen Bereichen der Technik werden Stanzstücke definierter Größe aus Materialien wie Glas, Keramik oder Kohlenstoff benötigt. Zur Herstellung von Fasern definierter Länge hat die Industrie geeignete Verfahren und Vorrichtungen entwickelt. So zeigt beispielsweise die US 4 638 934 eine Vorrichtung zum Kleinschneiden von Fiberglasmatten. Hierzu werden die Fiberglasmatten zunächst durch rollende Messer in Längsstreifen geschnitten. Diese Längsstreifen werden anschließend quer geschnitten. Während der Schneidevorgänge liegt die Fiberglasmatte auf einer elastischen Schneideunterlage.

Ein weiteres Verfahren und eine dazu geeignete Vorrichtung beschreibt die US 3 921 874. Hier werden lange Fäden des Ausgangsmaterials in eine Flüssigkeit getaucht, die anschließend durch Abkühlen verfestigt wird. Der gefrorene Faserstrang wird anschließend quer geschnitten. Nach dem Auftauen und Entfernen der Flüssigkeit liegen Kurzfasern vor.

Nachteilig bei diesen bekannten Verfahren und Vorrichtungen ist, dass zwischen dem ersten und dem zweiten Schneidvorgang Schlupf auftreten kann, so dass die Fertigprodukte voneinander abweichende Längen haben können. Das in der zweitgenannten US-Schrift offenbarte Verfahren hat drüber hinaus nur eine begrenzte Kapazität und ist somit für eine Großserienproduktion wenig geeignet.

Die DE 203 06 090 U1 beschreibt eine Vorrichtung zum rotativen Stanzen von Stanzstücken aus extrudierten Endlosprofilen, insbesondere zum Prägen und Lochen von aus einem Extruder kommenden Endlosprofilen, wobei jedoch Stanzrückstände übrig bleiben, welche die Produktivität des Verfahrens einschränken.

Eine gattungsbildende Vorrichtung wird in der US 104 442 A beschrieben.

Der vorliegenden Erfindung liegt die Aufgabe zugrunde, eine Vorrichtung der eingangs erwähnten Art derart weiter zu entwickeln, dass sie kostengünstig und flexibel betrieben werden kann.

Diese Aufgabe wird durch die Merkmale des Patentanspruchs 1 gelöst.

Weiterhin sollen für die Vorrichtung besonders vorteilhafte Verwendungen angegeben werden.

Diese Aufgabe wird durch die Merkmale des Patentanspruchs 15 gelöst.

Gemäß Anspruch 1 werden die Schneidstempel und die Schneidlöcher am Umfang der Werkzeugträger jeweils derart lückenlos abwechselnd angeordnet, dass das Flächengebilde in einem einzigen Arbeitsgang verlust- und reststückfrei zu Stanzstücken verarbeitet wird. Weil das Flächengebilde dann komplett in Stanzstücke umgesetzt wird, ist die Produktivität maximal und es verbleibt kein Restmaterial. Eine solche Vorgehensweise ist vor allem bei Flächengebilden von Vorteil, deren Herstellung kostspielig ist oder welche aus einem wertvollen Material wie beispielsweise Edelmetall bestehen. Da die Stanzstücke in einem einzigen Arbeitsgang hergestellt werden, ergibt sich weiterhin eine hohe Arbeitsgeschwindigkeit und damit eine hohe Produktivität.

Erfindungsgemäß weisen die Werkzeugträger Ringlamellen mit jeweils an ihrem Umfang lückenlos abwechselnd ausgebildeten Schneidstempeln und Schneidlöchern auf, die derart axial gestapelt und in Umfangsrichtung jeweils um eine Teilung gegeneinander versetzt sind, dass sich auch in Axialrichtung gesehen Schneidstempel und Schneidlöcher lückenlos abwechseln. Dann können bei Werkzeugverschleiß einzelne Ringlamellen einfach ausgewechselt werden, ohne dass das gesamte Werkzeug ersetzt werden muss. Die Dicke der Ringlamellen ist dann identisch mit der Breite der zu stanzenden Stanzstücke.

Durch die in den Unteransprüchen aufgeführten Maßnahmen sind vorteilhafte Weiterbildungen und Verbesserungen der im Anspruch 1 angegebenen Erfindung möglich.

Besonders bevorzugt sind die Schneidstempel und die Schneidlöcher derart ausgebildet, dass ausschließlich Stanzstücke mit identischer Geometrie und Größe ausgestanzt werden, beispielsweise rechteckförmige oder quadratische Stanzstücke.

Zur Realisierung einer höheren Produktivität können die Schneidstempel und die Schneidlöcher sowohl in Umfangsrichtung als auch in axialer Richtung gesehen am Umfang der Werkzeugträger schachbrettartig abwechselnd angeordnet sein. Dann werden in jeder Drehlage der Werkzeugträger mehrere Stanzstücke erzeugt.

Die Ringlamellen bestehen vorzugsweise aus Stahl, der insbesondere im Bereich der Schneidstempel und/oder Schneidlöcher gehärtet oder einsatzgehärtet und/oder mit einer verschleißarmen Schicht, beispielsweise aus Hartmetall beschichtet ist. Auch kommen wegen ihrer Verschleißbeständigkeit keramische Werkstoffe für die Ringlamellen in Frage, beispielsweise in Form einer Beschichtung im Bereich der Schneidstempel und/oder Schneidlöcher.

Besonders bevorzugt erstrecken sich Kanäle vom Innenumfang der Ringlamellen bis in die Schneidlöcher.

Gemäß einer Variante können in den Kanälen Auswerfer radial verschieblich geführt sein, wobei die Auswerfer bevorzugt T-förmige flächige Gebilde sind und einen im Schneidloch angeordneten, mit den Stanzstücken in Kontakt tretenden Querbalken, einen im Kanal geführten Schaft sowie einen aus dem Kanal nach radial innen hervorragenden Fußteil aufweisen. Weiterhin kann im Inneren der Werkzeugträger je wenigstens eine Walze derart exzentrisch angeordnet sein, dass sie abhängig von der Drehposition des Werkzeugträgers in Kontakt mit dem Fußteilen wenigstens eines Auswerfers tritt und diesen dadurch nach radial außen drängt, wodurch das im zugeordneten Schneidloch befindliche Stanzstücke ausgeworfen wird.

Zur radialen Betätigung der Auswerfer ist im Inneren wenigstens eines rotierenden Werkzeugträgers eine Walze derart lose gehalten, dass sie durch Schwerkraftwirkung in Kontakt mit den Fußteilen der Auswerfer treten kann. Alternativ kann eine solche innere Walze auch an einer festen Achse exzentrisch gelagert sein.

Gemäß einer weiteren Ausführungsform kann eine Druckerzeugungseinrichtung vorgesehen sein, welche wenigstens einige der Kanäle unter Druck eines Druckmittels setzt, wobei das Druckmittel bevorzugt Wasser beinhaltet. Zum Einspritzen des Druckmittels in die Kanäle kann beispielsweise wenigstens eine Düse vorgesehen sein, welche abhängig von der Drehposition des Werkzeugträgers mit einem Kanal in Flucht bringbar ist.

Die beschriebene Vorrichtung wird zum verlust- und reststückfreien Stanzen von Stanzstücken definierter Geometrie und Größe aus wenigstens Leder, Fasergelegen oder Folien verwendet, wobei die Fasergelege bevorzugt in Form von Gelegen, Geweben, Papier, non-wovens oder Filzen vorliegen und aus Natur-, Kunststoff-, Glas- oder Kohlenstofffasern mit und ohne Binder bestehen und die Folie beispielsweise aus Kunststoff, Metall oder expandiertem Graphit besteht.

Besonders vorteilhaft werden Fasergebilde, bevorzugt Kohlenstofffasern als Flächengebilde verwendet, die mit Zuschlagstoffen und/oder aushärtbarem Bindemittel versehen sind. Aus einem solchen, mit Kunstharz getränkten Faserstrang mit einer im wesentlichen parallel verlaufende Filamente beinhaltenden Faserlage, die ein unidirektionales Faserband ausbildet, können dann nach Abkühlen und Aushärten des Binders mit Hilfe der Vorrichtung Bandstücke oder Bündel definierter Länge und Breite verlustfrei hergestellt werden. Diese Bandstücke oder Bündel werden beispielsweise zur Herstellung kurzfaserverstärkter Werkstoffe sortenrein oder in Mischung von verschiedenen Längen oder Breiten eingesetzt. Für diese Art von Werkstoffen sind abhängig von den geforderten Werkstoffeigenschaften eine bestimmte Länge der Bandstücke oder Faserbündel wesentlich. Siebfraktionen gemahlener Fasern oder Faserbündel enthalten demgegenüber Fraktionen mit für den beabsichtigten Zweck ungeeigneten Faserlängen. Mit der Vorrichtung gemäß der Erfindung ist jedoch sichergestellt, dass die Verstärkungsfasern eine einheitliche Länge und Bündelstärke aufweisen. Für den Fall, dass Mischungen von Faserlängen (Faserlängenverteilung) gefordert sind, ist das Spektrum der Faserlängenverteilung oder der Bündeldickenverteilung ebenso gezielt einstellbar.

Weiterhin kann das Flächengebilde in Form von Blättern, einschichtig oder mehrschichtig vorliegen, wobei sich beim Stanzen von übereinander gestapelten Blättern eine gesteigerte Produktivität ergibt.

Besonders bevorzugt können mit der Vorrichtung Stanzstücke mit einer Dicke von 5 µm bis 5 mm, einer Breite von 0,1 mm bis 10 mm und einer Länge von 1 mm bis 100 mm hergestellt werden.

Genaueres geht aus der folgenden Beschreibung von Ausführungsbeispielen hervor.

### Zeichnung

Nachstehend sind Ausführungsbeispiele der Erfindung in der Zeichnung dargestellt und in der nachfolgenden Beschreibung näher erläutert. In der Zeichnung zeigt
- Fig. 1: eine entlang der Linie I aufgeschnittene Stirnansicht einer Vorrichtung zum rotativen Stanzen von Stanzstücken gemäß einer bevorzugten Ausführungsform der Erfindung;
- Fig.2: eine Seitenansicht der Vorrichtung von Fig.1;
- Fig.3: eine aufgeschnittene Stirnansicht einer Vorrichtung zum rotativen Stanzen von Stanzstücken gemäß einer weiteren Ausführungsform der Erfindung;
- Fig.4: eine Stirnansicht von Ringlamellen der Vorrichtung von Fig.3.

### Beschreibung der Ausführungsbeispiele

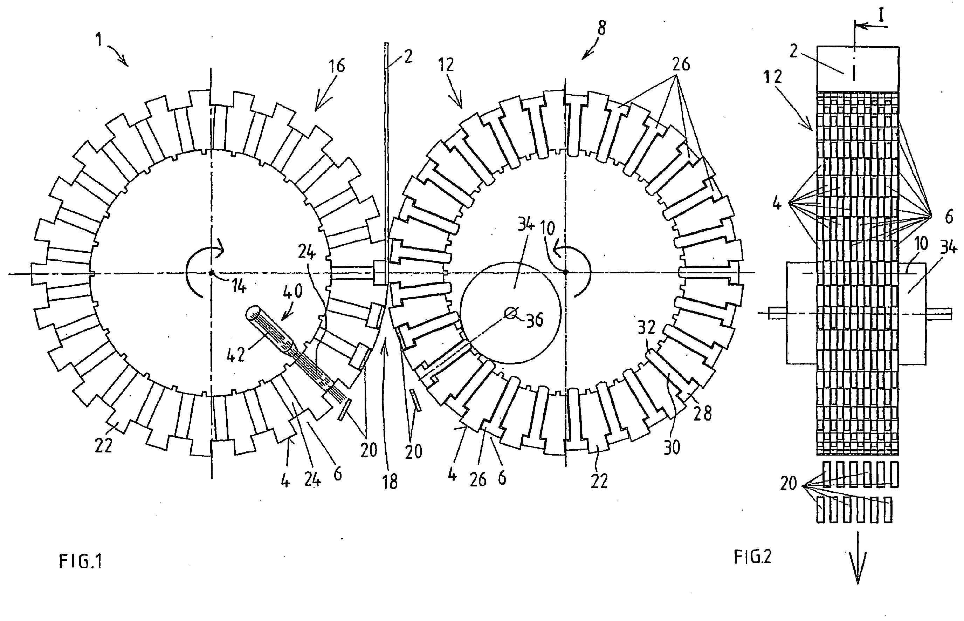

In Fig.1 ist mit der Bezugszahl 1 eine bevorzugte Ausführungsform einer Vorrichtung zum rotativen Stanzen von Stanzstücken definierter Geometrie und Größe aus einem Flächengebilde 2 gemäß der Erfindung bezeichnet.

Die Vorrichtung 1 beinhaltet ein mehrere Schneidstempel 4 und Schneidlöcher 6 aufweisendes Werkzeug 8 mit einem um eine erste Achse 10 rotierenden ersten Werkzeugträger 12 in Form einer Trommel und einen um eine zweite Achse 14 rotierenden zweiten, ebenfalls trommelförmigen Werkzeugträger 16. Dabei sind die erste Achse 10 und die zweite Achse 14 parallel, koplanar und mit Radialabstand zueinander derart angeordnet, dass zwischen dem ersten Werkzeugträger 12 und dem zweiten Werkzeugträger 16 ein radialer Abstand zur Bildung eines Durchtrittsspalts 18 für das Flächengebilde 2 vorhanden ist und durch gegenläufige Rotation der beiden Werkzeugträger 12, 16 ähnlich miteinander kämmender Zahnräder die Schneidstempel 4 passgenau in die Schneidlöcher 6 eindringen.

Beim Eindringen der Schneidstempel 4 in die Schneidlöcher 6 wird ein dem Querschnitt des Schneidstempels 4 bzw. des korrespondierenden Schneidlochs 6 entsprechendes Stanzstück 20 aus dem Flächengebilde 2 herausgestanzt und in das Schneidloch 6 gedrückt, wobei es dort anhängig von Toleranzen und dem elastischen Verhalten des Flächengebildematerials zu einem Kraftschluss zwischen dem Rand des Stanzstücks 20 und der Schneidlochwandung kommt, welcher die Tendenz hat, das Stanzstück 20in dem Schneidloch 6 zu halten.

Besonders bevorzugt sind die Schneidstempel 4 und die Schneidlöcher 6 derart ausgebildet, dass ausschließlich Stanzstücke 20 mit identischer Geometrie und Größe ausgestanzt werden, hier beispielsweise rechteckförmige Stanzstücke. Wie aus Fig.2 hervorgeht, sind die Schneidstempel 4 und die Schneidlöcher 6 sowohl in Umfangsrichtung als auch in axialer Richtung gesehen am Umfang der Werkzeugträger 12, 16 schachbrettartig abwechselnd und lückenlos angeordnet, so dass das in seiner Breite nicht über die Breite der Werkzeugträger 12 und 16 hinausgehende Flächengebilde 2 vollständig und rückstandslos in Stanzstücke 20 umgesetzt wird.

Die Werkzeugträger 12, 16 tragen jeweils identische Ringlamellen 22 mit an ihrem Umfang lückenlos abwechselnd ausgebildeten Schneidstempeln 4 und Schneidlöchern 6, die derart axial gestapelt und in Umfangsrichtung um jeweils eine Teilung gegeneinander versetzt sind, dass sich auch in Axialrichtung gesehen Schneidstempel 4 und Schneidlöcher 6 lückenlos abwechseln. Die Dicke der Ringlamellen 22 ist dann identisch mit der Breite der zu stanzenden Stanzstücke 20. Aus Anschauungsgründen sind in der Schnittsdarstellung von Fig.1 nur zwei miteinander kämmende Ringlamellen 22 der beiden Werkzeugträger 12, 16 dargestellt. Die Ringlamellen 22 können beispielsweise auf einer Nabe des betreffenden Werkzeugträgers 12, 16 einander axial kontaktierend aufgenommen sein und mit dieser beispielsweise über Verzahnungen zumindest drehfest verbunden sein.

Die Ringlamellen 22 bestehen vorzugsweise aus Stahl, der insbesondere im Bereich der Schneidstempel 4 und/oder Schneidlöcher 6 gehärtet oder einsatzgehärtet oder mit einer verschleißarmen Schicht, beispielsweise aus Hartmetall beschichtet ist. Auch kommen wegen ihrer Verschleißbeständigkeit keramische Werkstoffe für die Ringlamellen 22 in Frage, beispielsweise in Form einer Beschichtung im Bereich der Schneidstempel 4 und/oder Schneidlöcher 6.

Besonders bevorzugt erstrecken sich Kanäle 24 vom Innenumfang der Ringlamellen 22 bis in die Schneidlöcher 6.

Gemäß einer in Fig.1 rechts gezeigten Variante können in den Kanälen 24 der Ringlamellen 22 Auswerfer 26 radial verschieblich geführt sein, die bevorzugt T-förmige flächige Gebilde sind und einen im Schneidloch 6 angeordneten, mit den Stanzstücken 20 in Kontakt tretenden Querbalken 28, einen in dem Kanal 24 geführten Schaft 30 sowie einen aus dem Kanal 24 nach radial innen hervorragenden Fußteil 32 aufweisen. Weiterhin kann im Inneren des Ringlamellenpakets eine Walze 34 derart exzentrisch angeordnet sein, dass sie abhängig von der Drehposition des Werkzeugträgers 12 in Eingriff mit dem Fußteil 32 wenigstens eines Auswerfers 26 tritt und diesen dadurch nach radial außen drängt, wodurch das in dem zugeordneten Schneidloch 6 befindliche, dort durch Kraftschluss gehaltene Stanzstück 20 ausgeworfen wird, bevorzugt in Schwerkraftrichtung nach unten. Die Achse der Walze 34 ist dabei parallel zur Achse 10 des ersten Werkzeugträgers 12. Weiterhin hat die Walze 34 einen im Vergleich zum Innendurchmesser des Innenumfangs der Ringlamellen 22 kleineren Außendurchmesser.

Zur radialen Betätigung der Auswerfer 26 ist im Inneren des rotierenden Werkzeugträgers 12 gemäß der Ausführungsform von Fig.1 rechts die Walze 34 an einer festen Achse 36 exzentrisch gelagert, die bevorzugt in dem dem Durchtrittsspalt 18 in Drehrichtung nachgeordneten Quadranten des Werkzeugsträgers 12 angeordnet ist. Bedingt durch den Drehantrieb des Werkzeugträgers 12 und der raumfesten Drehlagerung der Walze 34 laufen während einer ganzen Umdrehung des Werkzeugträgers 12 die Fußteile 32 sämtlicher Auswerfer 26 auf der Walzenoberfläche auf und werden dadurch nach radial außen gedrängt, was ein Auswerfen der Stanzstücke 20 aus den Schneidlöchern 6 dieses Werkzeugträgers 12 bedingt. Durch die Wirkung der Schwerkraft, wenn die Auswerfer 26 in die beiden oberen Quadranten weitergedreht werden bzw. durch Eindringen der Schneidstempel 4 des anderen Werkzeugträgers 16 in die zugeordneten Schneidlöcher 6 werden die Auswerfer 26 wieder in ihre radial innere Ausgangslage zurück verschoben. Um ein Herausfallen der Auswerfer 26 aus den Kanälen 24 zu verhindern, können die Querschnitte der Fußteile 32 gegenüber den Schäften 30 erweitert sein.

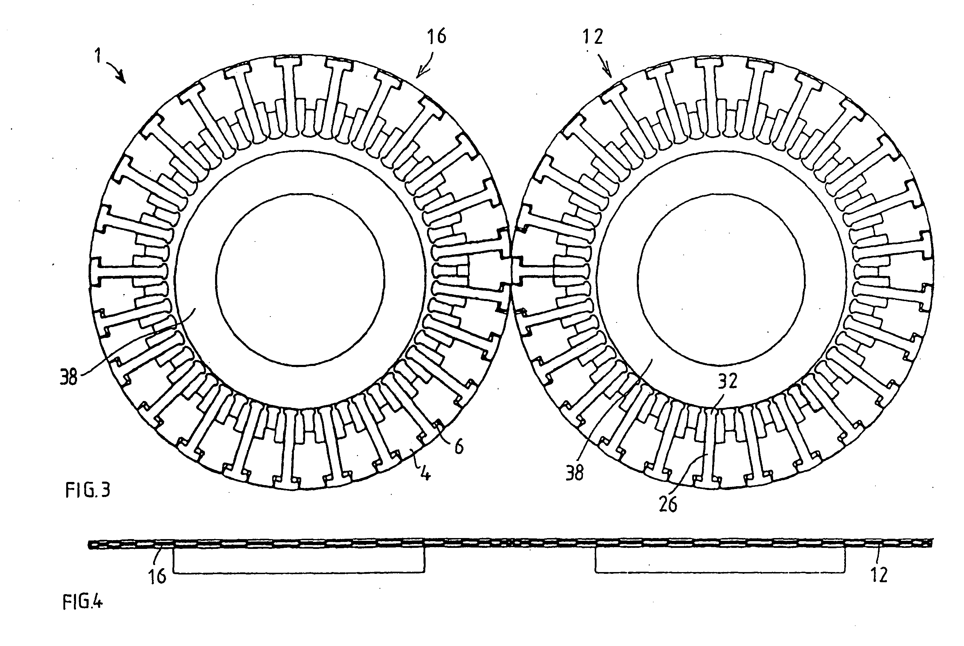

Gemäß einer weiteren Ausführungsform nach Fig.3 kann eine solche parallele Walze 38 auch lose im Inneren eines Werkzeugträgers 12, 16 gehalten sein, so dass diese durch Schwerkraftwirkung in Kontakt mit den Fußteilen 32 der Auswerfer 26 treten kann. Die Walze 38 hat wiederum einen im Vergleich zum Innendurchmesser des Innenumfangs der Ringlamellen 22 kleineren Außendurchmesser und läuft bedingt durch den Antrieb durch den Werkzeugträger 12, 16 ein Stück in Drehrichtung mit, um dann durch die Schwerkraftwirkung wieder in ihre untere Ausgangslage zurückzukehren bzw. um diese schwingend zu rollen, was aufgrund der dabei auftretenden dynamischen Kräfte eine radiale Betätigung der Auswerfer 26 erleichtert.

Gemäß einer weiteren, in Fig.1 links gezeigten Ausführungsform kann eine Druckerzeugungseinrichtung 40 vorgesehen sein, welche zum Auswerfen der Stanzstücke 20 wenigstens einen Kanal 24 von radial innen unter Staudruck eines Druckmittels setzt, wobei das Druckmittel bevorzugt Wasser beinhaltet. Zum Einspritzen des Druckmittels in den Kanal 24 kann beispielsweise wenigstens eine Düse 42 vorgesehen sein, welche abhängig von der Drehposition des Werkzeugträgers 16 mit einem Kanal 24 in Flucht bringbar ist. Im allgemeinen hängt daher ein Auswurf eines in einem Schneidloch 6 kraftschlüssig gehaltenen Stanzstücks 20 von der Drehlage des Werkzeugträgers 12, 16 ab.

Gemäß einer hier nicht gezeigten Ausführungsform kann in den Schneidlöchern 6 auch jeweils ein Federelement, beispielsweise in Form eines Gummistücks gehalten sein, welches beim Eindringen des Schneidstempels 4 in das betreffende Schneidloch 6 elastisch federnd zusammengepresst wird und sich nach dem Herausfahren des Schneidstempels 4 aus dem Schneidloch 6 wieder ausdehnt, wodurch das im Schneidloch 6 gehaltene Stanzstück 20 ausgeworfen wird.

Die beschriebene Vorrichtung wird zum verlust- und reststückfreien Stanzen von Stanzstücken 20 definierter Geometrie und Größe bevorzugt aus Leder, Fasergelegen oder Folien verwendet, wobei die Fasergelege bevorzugt in Form von Gelegen, Geweben, Papier, non-wovens oder Filzen vorliegen und aus Natur-, Kunststoff-, Glas- oder Kohlenstofffasern mit und ohne Binder bestehen und die Folie beispielsweise aus Kunststoff, Metall oder expandiertem Graphit besteht.

Besonders vorteilhaft werden Fasergebilde, bevorzugt Kohlenstofffasern als Flächengebilde 2 verwendet, die mit Zuschlagstoffen und/oder aushärtbarem Bindemittel versehen sind. Weiterhin kann das Flächengebilde in Form von Blättern, einschichtig oder mehrschichtig vorliegen.

Besonders bevorzugt können mit der Vorrichtung Stanzstücke 20 mit einer Dicke von 5 µm bis 5 mm, einer Breite von 0,1 mm bis 10 mm und einer Länge von 1 mm bis 100 mm hergestellt werden.

## Patentansprüche

1. Vorrichtung (1) zum rotativen Stanzen von Stanzstücken (20) definierter Geometrie und Größe aus einem Flächengebilde (2) mit einem mehrere Schneidstempel (4) und Schneidlöcher (6) aufweisenden Werkzeug (8), beinhaltend einen um eine erste Achse (10) rotierenden ersten Werkzeugträger (12) und einen um eine zweite Achse (14) rotierenden zweiten Werkzeugträger (16), wobei die erste Achse (10) und die zweite Achse (14) parallel und mit Radialabstand zueinander derart angeordnet sind, dass zwischen dem ersten Werkzeugträger (12) und dem zweiten Werkzeugträger (16) ein radialer Abstand zur Bildung eines Durchtrittsspalts (18) für das Flächengebilde (2) vorhanden ist und durch gegenläufige Rotation der Werkzeugträger (12, 16) die Schneidstempel (4) passgenau in die Schneidlöcher (6) eindringen, wobei die Schneidstempel (4) und die Schneidlöcher (6) am Umfang des ersten Werkzeugträgers (12) und am Umfang des zweiten Werkzeugträgers (16) jeweils derart lückenlos abwechselnd angeordnet sind, dass das Flächengebilde (2) in einem einzigen Arbeitsgang verlust- und reststückfrei zu Stanzstücken (20) verarbeitet wird, **dadurch gekennzeichnet, dass** die Werkzeugträger (12, 16) Ringlamellen (22) mit an ihrem Umfang lückenlos abwechselnd ausgebildeten Schneidstempeln (4) und Schneidlöchern (6) aufweisen, welche derart axial gestapelt und in Umfangsrichtung um jeweils eine Teilung gegeneinander versetzt sind, dass sich auch in Axialrichtung gesehen Schneidstempel (4) und Schneidlöcher (6) lückenlos abwechseln.

2. Vorrichtung nach Anspruch 1, **dadurch gekennzeichnet, dass** die Schneidstempel (4) und die Schneidlöcher (6) derart ausgebildet sind, dass ausschließlich Stanzstücke (20) mit identischer Geometrie und Größe ausgestanzt werden.

3. Vorrichtung nach Anspruch 1 oder 2, **dadurch gekennzeichnet, dass** die Schneidstempel (4) und die Schneidlöcher (6) sowohl in Umfangsrichtung als auch in axialer Richtung gesehen am Umfang der Werkzeugträger (12, 16) schachbrettartig abwechselnd angeordnet sind.

4. Vorrichtung nach einem der vorhergehenden Ansprüche, **dadurch gekennzeichnet, dass** die Dicke der Ringlamellen (22) identisch mit der Breite der zu stanzenden Stanzstücke (20) ist.

5. Vorrichtung nach Anspruch 4, **dadurch gekennzeichnet, dass** sich vom Innenumfang der Ringlamellen (22) Kanäle (24) bis in die Schneidlöcher (6) erstrecken.

6. Vorrichtung nach Anspruch 5, **dadurch gekennzeichnet, dass** in den Kanälen (24) Auswerfer (26) radial verschieblich geführt sind.

7. Vorrichtung nach Anspruch 6, **dadurch gekennzeichnet, dass** die Auswerfer (26) T-förmige flächige Gebilde sind und einen im Schneidloch (4) angeordneten, mit den Stanzstücken (20) in Kontakt tretenden Querbalken (28), einen im Kanal (24) geführten Schaft (30) sowie einen aus dem Kanal (24) nach radial innen hervorragenden Fußteil (32) aufweisen.

8. Vorrichtung nach Anspruch 7, **dadurch gekennzeichnet, dass** die Querschnitte der Fußteile (32) der Auswerfer (26) gegenüber den Schäften (30) erweitert sind.

9. Vorrichtung nach Anspruch 7 oder 8, **dadurch gekennzeichnet, dass** im Inneren der Werkzeugträger (12, 16) je wenigstens eine Walze (34; 38) derart exzentrisch angeordnet ist, dass sie abhängig von der Drehposition des Werkzeugträgers (12, 14) in Kontakt mit dem Fußteil (32) wenigstens eines Auswerfers (26) tritt und diesen dadurch nach radial außen drängt.

10. Vorrichtung nach Anspruch 9, **dadurch gekennzeichnet, dass** im Inneren wenigstens eines rotierenden Werkzeugträgers (12, 14) eine Walze (38) derart lose gehalten ist, dass sie durch Schwerkraftwirkung in Kontakt mit den Fußteilen (32) der Auswerfer (26) tritt.

11. Vorrichtung nach Anspruch 9, **dadurch gekennzeichnet, dass** im Inneren wenigstens eines rotierenden Werkzeugträgers (12) eine Walze (34) an einer festen Achse (36) exzentrisch gelagert ist.

12. Vorrichtung nach einem der Ansprüche 5 bis 11, **dadurch gekennzeichnet, dass** eine Druckerzeugungseinrichtung (40) vorgesehen ist, welche wenigstens einige der Kanäle (24) unter Druck eines Druckmittels setzt.

13. Vorrichtung nach Anspruch 12, **dadurch gekennzeichnet, dass** das Druckmittel Wasser beinhaltet.

14. Vorrichtung nach Anspruch 12 oder 13, **dadurch gekennzeichnet, dass** wenigstens eine Düse (42) vorgesehen ist, welche abhängig von der Drehposition des Werkzeugträgers (16) mit wenigstens einem Kanal (24) in Flucht bringbar ist, um das Druckmittel zuzuführen.

15. Verwendung der Vorrichtung (1) nach einem der vorhergehenden Ansprüche zum verlust- und reststückfreien Stanzen von Stanzstücken (20) definierter Geometrie und Größe aus wenigstens Leder, Fasergelegen oder Folien, wobei die Fasergelege in Form von Gelegen, Geweben, Papier, non-wovens oder Filzen vorliegen und aus Natur-, Kunststoff-, Glas- oder Kohlenstofffasern mit und ohne Binder bestehen und die Folie aus Kunststoff, Metall oder expandiertem Graphit besteht.

16. Verwendung der Vorrichtung (1) nach Anspruch 15 zum verlust- und reststückfreien Stanzen von Stanzstücken (20) aus Endlosmaterial, Bändern (2) oder Blättern, einschichtig oder mehrschichtig.

17. Verwendung der Vorrichtung nach Anspruch 15 oder 16 zur Herstellung von Stanzstücken (20) definierter Geometrie und Größe mit einer Dicke von 5 µm bis 5 mm, einer Breite von 0,1 mm bis 10 mm und einer Länge von 1 mm bis 100 mm.

## Claims

1. An apparatus (1) for rotational stamping of stamped blanks (20) with a defined geometry and size from a flat material (2), wherein the apparatus comprises a tool (8), including plural cutting dies (4) and cutting holes (6), and comprises a first tool support (12) rotating about a first axis (10) and a second tool support (16) rotating about a second axis (14), wherein the first axis (10) and the second axis (14) are disposed in parallel and at a radial distance from one another, so that a radial offset for forming a pass-through gap (18) for the flat material (2) is provided between the first tool support (12) and the second tool support (16), and the cutting dies (4) penetrate the cutting holes (6) precisely fitting through counteracting rotation of the tool supports (12, 16), wherein the cutting dies (4) and the cutting holes (6) are disposed respectively on the circumference of the first tool support (12) and on the circumference of the second tool support (16) in a seamless and alternating pattern, so that the flat material (2) is processed into stamped blanks (20) in a single step without lost or scrap pieces, wherein the tool supports (12, 16) comprise annular ribs (22), comprising cutting dies (4) and cutting holes (6), configured on their circumferences in a seamless and alternating pattern, wherein the cutting dies and cutting holes are stacked in axial direction and respectively offset with respect to one another by a pitch, so that the cutting dies (4) and the cutting holes (6) also alternate in a seamless pattern in axial direction,

2. The apparatus according to claim 1, wherein the cutting dies (4) and the cutting holes (6) are configured, so that only stamped blanks (20) with identical geometry and size are stamped out.

3. An apparatus according to claim 1 or 2, wherein the cutting dies (4) and the cutting holes (6) are disposed in an alternating checkerboard pattern on the circumferences of the tool supports (12, 16) in circumferential direction and also in axial direction.

4. The apparatus according to one of the preceding claims, wherein the thickness of the annular ribs (22) is identical to the width of the stamped blanks (20) to be stamped.

5. The apparatus according to claim 4, wherein channels (24) extend from the inner circumferences of the annular ribs (22) into the cutting holes (6).

6. The apparatus according to claim 5, wherein ejectors (26) are guided in the channels (24), so they are movable in radial direction.

7. The apparatus according to claim 6, wherein the ejectors (26) are T-shaped flat elements, comprising a transversal bar (28), disposed in the cutting hole (6) and contacting the stamped blanks (20), a shaft (30) guided in a channel (24), and a base portion (32), radially protruding in inward direction from the channel (24).

8. The apparatus according to claim 7, wherein the cross sections of the base portions (32) of the ejectors (26) are expanded relative to the shafts (30).

9. The apparatus according to claims 7 or 8, wherein at least one respective roller (34, 38) is eccentrically disposed in the interiors of the tool supports (12, 16), so that it contacts the base portion (32) of at least one ejector (26), depending on the rotation position of the tool support (12, 16), thus forcing the ejector in radial outward direction.

10. An apparatus according to claim 9, wherein a roller (38) is loosely supported in the interior of at least one rotating tool support (12, 16), so that it contacts the base portions (32) of the ejectors (26) due to gravity.

11. An apparatus according to claim 9, wherein a roller (34) is eccentrically supported at a fixed axis (36) in the interior of at least one rotating tool support (12).

12. An apparatus according to one of the claims 5 through 11, wherein a pressure generation apparatus (40) is provided, which pressurizes at least some of the channels (24) through a pressure means.

13. An apparatus according to claim 12, wherein the pressure means comprises water.

14. An apparatus according to claim 12 or 13, wherein at least one nozzle (42) is provided, which can be aligned with at least one channel (24), depending on the rotation position of the tool support (16), in order to feed the pressure means.

15. A method for using the apparatus (1) according to one of the preceding claims in order to stamp stamped blanks (20) of a defined geometry and size, at least from leather, fibrous layups or foils, wherein the fibrous layups are provided in the form of layups, cloths, paper, nonwoven materials or felts, and are comprised of natural, synthetic, glass or carbon fibers, with and without binder, and the foil is comprised of plastic, metal or expanded graphite, without lost or scrap pieces.

16. The method for using the apparatus (1) according to claim 15 for stamping stamped blanks (20) from endless materials, tapes or sheets in one layer or in plural layers, without lost or scrap pieces.

17. A method for using the apparatus according to claim 15 or 16 for producing stamped blanks (20) of a defined geometry and size, comprising a thickness of 5 µm to 5 mm, a width of 0.1 mm to 1 mm, and a length of 1 mm to 100 mm.

## Revendications

1. Dispositif (1) pour la découpe rotative de pièces découpées (20) de géométrie et de grandeur définie dans une structure en surface (2) avec un outil (8) qui présente plusieurs poinçons de découpage (4) et trous de découpage (6), contenant un premier porte-outil (12) rotatif autour d'un premier axe (10) et un second porte-outil (16) rotatif autour d'un second axe (14), le premier axe (10) et le second axe (14) étant placés parallèlement l'un à l'autre et avec un écart radial de telle manière qu'il existe un écart radial entre le premier porte-outil (12) et le second porte-outil (16) pour former une fente de passage (18) pour la structure en surface (2) et que les poinçons de découpage (4) pénètrent avec une prévision parfaite dans les trous de découpage (6) par rotation contraire des porte-outils (12, 16), les poinçons de découpage (4) et les trous de découpage (6) étant placés respectivement sur la périphérie du premier porte-outil (12) et sur la périphérie du second porte-outil (16) en alternance sans vides de telle manière que la structure en surface (2) est transformée en pièces découpées (20) en une seule opération sans perte et sans pièces restantes, **caractérisé en ce que** les porte-outils (12, 16) présentent des lamelles annulaires (22) avec des poinçons de découpage (4) et des trous de découpage (6) configurés en alternance sans vides sur leur périphérie, lamelles qui sont empilées dans le sens axial et qui sont décalées les unes par rapport aux autres dans lé sens de la circonférence de respectivement une graduation de telle manière que les poinçons de découpage (4) et les trous de découpage (6) alternent sans vides, ceci étant vu également dans le sens axial.

2. Dispositif selon la revendication 1, **caractérisé en ce que** les poinçons de découpage (4) et les trous de découpage (6) sont configurés de telle manière que seulement des pièces découpées (20) de géométrie et de grandeur identique sont découpées.

3. Dispositif selon la revendication 1 ou 2, **caractérisé en ce que** les poinçons de découpage (4) et les trous de découpage (6) sont placés en alternance en damier sur la périphérie des porte-outils (12, 16), ceci étant vu aussi bien dans le sens de la circonférence que dans le sens axial.

4. Dispositif selon l'une des revendications précédentes, **caractérisé en ce que** l'épaisseur des lamelles annulaires (22) est identique à la largeur des pièces découpées (20) à découper.

5. Dispositif selon la revendication 4, **caractérisé en ce que** des canaux (24) s'étendent de la périphérie intérieure des lamelles annulaires (22) jusque dans les trous de découpage (6).

6. Dispositif selon la revendication 5, **caractérisé en ce que** des éjecteurs (26) sont guidés déplaçables radialement dans les canaux (24).

7. Dispositif selon la revendication 6, **caractérisé en ce que** les éjecteurs (26) sont des structures en surface en forme de T et qu'ils présentent une traverse (28) placée dans un trou de découpage (4), entrant en contact avec les pièces découpées (20), une tige (30) guidée dans le canal (24) ainsi qu'une partie de base (32) qui fait saillie hors du canal (24) vers l'intérieur dans le sens radial.

8. Dispositif selon la revendication 7, **caractérisé en ce que** les sections des parties de base (32) des éjecteurs (26) sont élargies par rapport aux tiges (30).

9. Dispositif selon la revendication 7 ou 8, **caractérisé en ce qu'**au moins un cylindre (34 ; 38) est placé dans l'intérieur des porte-outils (12, 16) de manière excentrée de telle manière qu'en fonction de la position de rotation du porte-outil (12, 14) il entre en contact avec la partie de base (32) d'au moins un éjecteur (26) et pousse donc celui-ci vers l'extérieur dans le sens radial.

10. Dispositif selon la revendication 9, **caractérisé en ce qu'**un cylindre (38) est maintenu lâche à l'intérieur d'au moins un porte-outil rotatif (12, 14) de telle manière qu'il entre en contact avec les parties de base (32) des éjecteurs (26) par l'effet de la pesanteur.

11. Dispositif selon la revendication 9, **caractérisé en ce qu'**un cylindre (34) est positionné excentré sur un axe fixe (36) dans l'intérieur d'au moins un porte-outil rotatif (12).

12. Dispositif selon l'une des revendications 5 à 11, **caractérisé en ce qu'**il est prévu un dispositif de génération de pression (40) qui met au moins quelques uns des canaux (24) sous pression d'une substance sous pression.

13. Dispositif selon la revendication 12, **caractérisé en ce que** la substance sous pression contient de l'eau.

14. Dispositif selon la revendication 12 ou 13, **caractérisé en ce qu'**il est prévu au moins une tuyère (42) qui, en fonction de la position de rotation du porte-outil (16), peut être mise en alignement avec au moins un canal (24) pour amener la substance sous pression.

15. Utilisation du dispositif (1) selon l'une des revendications précédentes pour la découpe sans perte et sans pièces restantes de pièces découpées (20) de géométrie et de grandeur définie à partir d'au moins du cuir, des mats de fibres ou des feuilles, les mats de fibres existant sous forme de mats, de tissus, de papier, de non-tissés ou de feutres et consistant en fibres naturelles, synthétiques, en fibres de verre ou de carbone avec et sans liant et la feuille étant constituée par du plastique, du métal ou du graphite expansé.

16. Utilisation du dispositif (1) selon la revendication 15 pour la découpe sans perte et sans pièces restantes de pièces découpées (20) en matériau sans fin, en bandes (2) ou en feuilles, à une ou à plusieurs couches.

17. Utilisation du dispositif selon la revendication 15 ou 16 pour la fabrication de pièces découpées (20) de géométrie et de grandeur définie avec une épaisseur de 5 µm à 5 mm, une largeur de 0,1 mm à 10 mm et une longueur d'1 mm à 100 mm.
